# EUROPEAN PATENT APPLICATION

(11) **EP 3 332 812 A1**
(43) Date of publication of application: **13.06.2018**
(21) Application number: 16202754.4
(22) Date of filing: 07.12.2016
(51) Int. Cl.: A61K 47/56, A61K 47/54, A61K 47/69, A61K 9/107, A61K 31/704, A61P 35/00

(54) **NUCLEIC ACID-BASED ASSEMBLY AND USE OF THE ASSEMBLY IN CANCER THERAPY**

(71) Applicant: Rheinische Friedrich-Wilhelms-Universität Bonn, 53113 Bonn (DE)
(72) Inventor: FAMULOK, Michael, 53175 Bonn (DE); PRUSTY, Deepak, 53129 Bonn (DE); ADAM, Volker, 53123 Bonn (DE); IRSEN, Stephan, 53225 Bonn (DE)
(74) Representative: Michalski Hüttermann & Partner Patentanwälte mbB

(57) **Abstract**

The present invention relates to a nucleic acid-based assembly comprising: at least one nucleic acid aptamer, and at least one nucleic acid motif designed to physically capture a drug, wherein the nucleic acid motif comprises one or more photo-responsive moieties that effect the release of the drug upon irradiation, wherein the aptamer and the nucleic acid motif each are covalently linked to one or more lipids, wherein the lipid-modified aptamer and nucleic acid motif form the assembly through noncovalent interaction. The invention further relates to use of the nucleic acid-based assembly in the treatment of cancer.

## Description

The present invention relates to aptamer-based drug-delivery systems and their use in cancer therapy.

There is a compelling demand for improvements in the effectiveness in both the transport and specific release of therapeutic molecules. A powerful approach is the use of aptamer-based tumor targeting systems in combination with controlled release of active therapeutics through physiochemical responses to external stimuli such as pH, light, or chemicals. Due to their advantages over other targeting reagents such as easy synthesis, low immunogenicity, and high target affinity, DNA aptamers have opened up new opportunities for cellular targeting and have been selected against various cancer types. However, aptameric molecular nanocarriers are often limited by inefficient cellular uptake and short cytoplasmic half-life as they are naturally susceptible to nuclease-mediated degradation.

Progress has been made to improve serum half-life and cell internalization efficacy by functionalizing nanocarriers with aptamers that target specific surface proteins such as polymeric nanoparticles, liposomes, aptamer-drug conjugates, aptamer-antibody conjugates, and aptamer-functionalized quantum dots. However, the majority of these approaches entailed significant trade-offs between complicated assembly, suboptimal size, limited payload capacity, and some of them show insufficient serum stability and cell internalization efficacy. In case of aptamer-drug conjugates, covalent linking of targeting units to cytotoxic agents is limited by the concern that the attachment may alter their biological activity.

It is further known to employ a native cell-targeting aptamer that was modified by additional nucleobases for drug intercalation as a dual factor for cell targeting and, simultaneously, as a cargo for drug transport. US 9163048 B2 describes a multifunctional nucleic-acid-based anticancer drug prepared by physically capturing an anticancer drug in a linear nucleic acid having a thiol group at the 5'-end, and chemically binding gold nanoparticles and a nucleic acid aptamer. The multi-functional nucleic acid-based anti-cancer drug uses A10 aptamer to achieve high targeting properties and high-concentration anti-cancer drugs and gold nanoparticles to enable dual therapy of thermal and chemical therapy. Yet, there is an inherent limitation to broader applicability for such architectures, especially when extended to other aptameric platforms for targeting different cell types, even a minor modification of the aptamer sequence with a drug loading unit might result in significant disruption of binding affinity. Moreover, demanding manufacturing processes are needed to provide such multifunctional nucleic-acid-based anticancer drugs. Further issues remain the triggered release of the active drug, the obstacles of tumor penetration and low structural stability.

Therefore, the object underlying the present invention was to provide a delivery system that facilitates manufacture. Further, the uptake efficiency should be improved.

The problem is solved by a nucleic acid-based assembly comprising:
- at least one nucleic acid aptamer, and
- at least one nucleic acid motif designed to physically capture a drug, wherein the nucleic acid motif comprises one or more photo-responsive moieties that effect the release of the drug upon irradiation,
wherein the aptamer and the nucleic acid motif each are covalently linked to one or more lipids, wherein the lipid-modified aptamer and nucleic acid motif form the assembly through noncovalent interaction.

Surprisingly it was found that the lipid-functionalized aptamer and nucleic acid motif provide a highly versatile nano-level assembly, which forms by spontaneous self-assembly by simple mixing of the lipid-modified aptamer and nucleic acid motif. The invention advantageously provides a multi-functional assembly that can encompass a cell-targeting aptamer unit and a separate nucleic acid motif with drug loading sites, where both are held together within a single nano-size scaffold through noncovalent interactions. The design of the assembly allows using a large variety of lipid-modified aptamers or molecules that can self-assemble into a functional nano-size assembly. This provides for a highly versatile applicability. The assembly further provides good nuclease stability, and high target binding affinity and cellular uptake. These features advantageously allow a wide applicability for the simultaneous delivery of a variety of different regulatory molecules, such as antagomirs, small interfering RNAs, microRNAs, and pharmaceutical drugs with high specificity and efficiency.

It could be shown that the lipid-modified aptamer and nucleic acid motif self-assemble to form hybrid heterogeneous nanoconstructs of spherical geometry when the lipid modifications are present. The lipid-modified aptamer and nucleic acid motif can form an assembly of spherical or essentially spherical geometry, particularly a hybrid micellar construct. The size of the assembly may result from the physico-chemical properties of the aptamer and the nucleic acid motif, or from structural differences, or both. The size of the assembly further may depend on the lipid. Using biocompatible lipids the size of the assembly advantageously may be that of a nano-level structure. The assembly may have an average diameter in a range from ≥ 15 nm to ≤ 50 nm, or in a range from ≥ 20 nm to ≤ 40 nm. The assembly preferably has an average diameter in a range from ≥ 20 nm to ≤ 40 nm. The term "average diameter" refers to the average value of all diameters or arithmetically averaged diameters, relative to all particles.

A self-assembled aggregation advantageously can be effected by simple mixing of the lipid-modified aptamer and nucleic acid motif. The lipid-modification not only provides for self-assembled aggregation of micellar nanostructures, but chemically linking the aptamer and the nucleic acid motif to biocompatible lipids also can improve uptake efficiency and reduce nuclease-mediated degradation of the assembly in a cell. The assembly, which is held together through noncovalent interaction, further showed good integrity. It could be shown that the self-aggregated nanoconstructs were stabilized in aqueous solution through hydrophobic interaction of the lipids. Such self-assembled structures even offer an unprecedented degree of control over the ratio of different functional domains based on the therapeutic requirements.

As used herein, the term "at least one" nucleic acid aptamer or nucleic acid motif particularly refers to the species of the aptamer and nucleic acid motif, and is not intended to limit the number of aptamer molecules and nucleic acid motif molecules comprised in the assmbly. The assembly may comprise a multitude of each of aptamer and nucleic acid motif.

The present invention will be further described in connection with various embodiments and other aspects. They may be combined freely unless the context clearly indicates otherwise.

The lipid may be an aliphatic hydrocarbon or fatty acid, such as, for example, C₈-C₂₄-alkanes, C₈-C₂₄-alkenes, and C₈-C₂₄-alkynes, and particularly may be selected from saturated and unsaturated fatty acids. Preferably, the lipid-modification may be the covalent binding to a C₈₋₂₄ saturated or unsaturated fatty acid chain. The saturated or unsaturated fatty acid chain preferably comprises 10 to 18 carbon atoms, or 12 to 16 carbon atoms. In embodiments, the lipid is selected from the group comprising C₁₂, C₁₄, C₁₆ and C₁₈ saturated and unsaturated fatty acid chains. Biocompatible lipids advantageously can improve uptake efficiency of the assembly. Further, fatty acid chains provide an effectively linear lipophilic chain, which supports the formation of regular micelles. In most preferred embodiments the lipid-modification is provided by C₁₂-lipid chains. It was observed that the C₁₂ lipid modification attached to the 5'-end of the aptamer induced self-aggregation of spherical micellar nanoconstructs at a concentration above the critical micelle concentration in aqueous solution.

The lipids may be covalently linked directly with the nucleic acids of the aptamer or the nucleic acid motif. Lipid-modified oligo(deoxy)nucleotides are commercially available. Or lipid modifications can be synthezised chemically. Nucleotides synthesized with a thio group can be coupled to maleimide-functionalized lipids, while nucleotides bearing a carboxylic acid or amine functionality can be coupled to an amine- or carboxylic acid-functionalized lipid. In embodiments, lipid-modified aptamers and nucleic acid motifs may be synthesized using lipid-modified phosphoramidites with a C₁₂-lipid chain incorporated at the 5-position of, for example, uridine-phosphoramidite. These modified bases may be attached to the nucleic acids, thereby introducing lipid tails into the aptamer and/or the nucleic acid motifs. Preferred is a terminal lipid modification of the aptamer and/or nucleic acid motif at the 3' and/or 5'-end. A terminal modification has the advantage of supporting the formation of spherical micellar structures. Further, the synthesis of a lipid-modified nucleic acid sequence that is modified only terminally can be carried out with commercially available monomers, and synthesis protocols known in the prior art can be used. A lipid modification preferably is provided at the 5'-end of the aptamer or the nucleic acid motif. The coupling of lipid-modified amidites to the 5'-end of nucleic acids can easily be incorporated when the nucleic acid is synthesized, for example by the process of amidite chemistry. Preferably, the lipid modification is provided at the 5'-end of the nucleic acid by specially modified phosphoramidites following a phosphoramidite process for the synthesis of the nucleic acid. For example, 5-(1-dodecynyl)-modified-2'-deoxyuridine-phosphoramidite groups may be used.

The aptamer and the nucleic acid motif each are covalently linked to one or more lipids. In embodiments, the lipid-modified aptamer and/or nucleic acid motif are covalently linked to 2 to 6, preferably to 3, 4 or 5 lipids. The lipids may be covalently linked directly with the respective nucleic acid. Preferably, four lipids, particularly C₁₂-lipid chains, may be attached to the 5'-end of the aptamer and/or the nucleic acid motif. In embodiments, four C₁₂-lipid modified deoxyuridine residues may be attached to the 5'-end. It could be shown that both, the aptamer and the nucleic acid motif, self-assemble to form hybrid heterogeneous nanoconstructs of spherical geometry when the lipid modifications are present. In preferred embodiments, the aptamer and/or the nucleic acid motif comprise a terminal lipid modification, preferably in a range from 2 to 6, more preferably 3, 4 or 5 lipids attached to the 5'-end. The ability to form nanoconstructs due to lipidation, and the lipidation providing for efficient uptake into cancer cells are important advantages of the assembly, particularly supporting an endocytotic uptake mechanism.

The nucleic acid-based assembly comprises at least one nucleic acid aptamer. As used herein, the term "nucleic acid aptamer" refers to an oligonucleotide molecule that binds to a specific target molecule. Aptamers usually bind to the target with high affinity, for example in the low nano molar range. The aptamer can be provided in the form of a single-stranded DNA or RNA molecule, or chemically modified versions thereof, such as 2'-F 2'-deoxy modified aptamers. Preferably, the aptamer comprises a deoxyribonucleotide and/or a 2'-F 2'-deoxy moified sequence. These can exhibit better stability. The nucleic acid aptamer provides a cell-targeting property to the assembly. A high targeting property advantageously minimizes effects of the drug on non-target cells.

In preferred embodiments, the aptamer targets the hepatocyte growth factor receptor (HGFR), also called cMet. HGFR is a transmembrane receptor protein that is overexpressed on the surface of numerous solid tumors. The ability to bind extracellular cMet by the aptamer moieties is a further feature supporting efficient uptake into cancer cells. In a preferred embodiment, the aptamer comprises the nucleotide sequence 5'-TGGATGGTAGCTCGGTCGGGGTGGG-TGGGTTGGCAAGTCT-3' (SEQ ID NO: 1). Aptamers comprising the SEQ ID NO: 1 bind with high specificity and affinity to the hepatocyte growth factor receptor, particularly with nano molar affinity. Also usable are aptamers targeting a cancer biomarker protein, or a protein that is expressed on a target cell. Usable aptamers, for example, are aptamers that specifically bind to single cancer cell types, i.e. to cancer cells (over)expressing proteins specific for a certain tumor on the cells surface. Various cancer-specific aptamers are known or are accessible by the systematic evolution of ligands by exponential enrichment (SELEX) process, particularly via cell-SELEX approach using whole live cells as targets to select aptamers for cell recognition.

A lipid-modified *anti*-cMet aptamer, particularly comprising the sequence SEQ ID NO: 1, may contain four C₁₂-lipid-functionalized dU-phosphoramidites at the 5'-end. It was found that lipidation of a cMet-binding aptamer improves efficient uptake into cancer cells. It is assumed that advantageous features for efficient uptake into cancer cells are the ability to bind extracellular cMet by the aptamer, and the ability to form nanoconstructs due to the lipidation.

The nucleic acid-based assembly comprises at least one nucleic acid motif designed to physically capture a drug. In preferred embodiments, the nucleic acid motif is a 5'-GC rich oligodeoxynucleotide that forms one or more hairpin loops that can intercalate the drug. Such 5'-GC-rich hairpin oligodeoxynucleotide particularly are able to intercalate and transport planar aromatic therapeutic agents such as doxorubicin. The nucleic acid motif preferably contains several, for example three or four GC rich hairpins. Integrating multiple GC-rich hairpin-duplex motifs affords several folds of loading of drug into a single nano scaffold, thereby enhancing the payload capacity in comparison to a monomeric aptamer.

The nucleic acid motif comprises one or more photo-responsive moieties that effect the release of the drug upon irradiation. Preferably, the nucleic acid motif comprises at least one photo-responsive moiety located within the base-pairing regions into which the drug intercalates, particularly within the hairpin region or regions. As used herein, the term "photo-responsive" moiety refers to an organic group, which undergoes isomerization and conformational change induced by irradiation, for example with visible light, ultraviolet light, or X-ray. A preferred photo-responsive moiety is an azobenzene group, a molecule with two phenyl rings joined by an azo linkage. Azobenzene can reversibly change trans/cis conformation upon exposure to irradiation energy. The photo induced transformation of photo-responsive molecules such as azobenzene derivatives incorporated into oligodeoxynucleotide backbones leads to a molecular motion which causes a structural change and thus is able to reversibly open and close oligodeoxynucleotide duplexes upon irradiation. Preferred azobenzene derivatives are 2'-methylazobenzene, particularly 2',6'-dimethylazobenzene (DMAB). Preferably, the nucleic acid motif contains several dimethylazobenzene moieties, for example 2 to 6, preferably 3, 4 or 5 dimethylazobenzene moieties.

In particular, azobenzenes tethered on D-threoninol allows incorporation of the azobenzenes into oligodeoxynucleotide backbones. The nucleic acid motif may contain one or more, for example 2 to 6, preferably 3, 4 or 5, particularly four, 2',6'-dimethylazobenzene-D-threoninol residues. In preferred embodiments, the nucleic acid motif comprises the nucleotide sequence 5'-GCNGCGNCTCNGCGNCGATTATTACGCGCGAGCGCGC-3' (SEQ ID NO: 2),
wherein N is a 2',6'-dimethylazobenzene-D-threoninol residue. The assembly thus can advantageously be provided with an inbuilt photo-regulated release mechanism for the drug. The nucleic acid motif preferably comprises the sequence SEQ ID NO: 2 with 4 DMAB moieties introduced into the sequence, and four lipid-chains attached to the 5'-end.

As used herein, the term "drug" refers to any substance other than food, that causes a physiological change in the body. A drug may be a regulatory molecule, such as antagomirs, small interfering RNAs, microRNAs, and pharmaceutical drugs. In preferred embodiments, the drug is an anti-cancer drug, preferably doxorubicin. Doxorubicin is a most potent and widely used chemotherapeutic. The IUPAC name of doxorubicin is (7*S*,9*S*)-7-[(2*R*,4*S*,5*S*,6*S*)-4-amino-5-hydroxy-6-methyloxan-2-yl]oxy-6,9,11-trihydroxy-9-(2-hydroxyacetyl)-4-methoxy-8,10-dihydro-7*H*-tetracene-5,12-dione. Doxorubicin is a planar aromatic molecule that is able to intercalate into oligodeoxynucleotides such as of (SEQ ID NO: 2).

In embodiments, the nucleic acid-based assembly may comprise a second aptamer, particularly a second nucleic acid aptamer. A "second aptamer" as used herein refers to a second species of aptamer and is not intended to limit the number of aptamer molecules comprised in the assmbly. A preferred second aptamer is an aptamer targeting a cancer biomarker protein, or a protein that is (over)expressed on a target cell such as a cancer cell.

In embodiments, the drug is released upon irradiation by visible light, ultraviolet light, or X-ray. Visible light may have a wavelength in a range from 380 nm to 435 nm. Visible light may cause no or only limited harm to the irradiated tissue. Suitable UV light irradiation may have a wavelength in a range from 320 nm to 400 nm. A usable UV wavelength is 365 nm. It could be shown that after UV irradiation, most of the intercalated drug was released from the assembly and subsequently transferred in the cell nuclei. UV light triggering with a penetration depth of light of a few millimeters may be sufficient for use with some melanoma. For other cancer types, azobenzene photo switches that isomerize with red light may be usable. Alternatively, fiber optic endoscopy might direct UV light to potential tumor sites deeper inside the body. Suitable X ray irradiation may have a wavelength in a range from 630 nm to 660nm. X ray irradiation may not only release the drug, but also itself have a therapeutic effect on the cancer cells.

The lipid-mediated facile assembly of the aptamer and nucleic acid motifs into hybrid nanoconstructs further advantageously allows for a precise control of the aptamer density on the surface of the assembly simply by mixing the cell-targeting aptamer with the drug-carrying nucleic acid motif in different ratios. In embodiments, the lipid-modified aptamer and nucleic acid motif are present in the assembly in a ratio in a range from ≥ 1:2 to ≤ 3:2, preferably in a 1:1 ratio. Such ratios can provide for an assembly providing most advantageous balance between high target affinity and internalization efficiency and therapeutically effective results based on drug carrying efficiency.

The assembly can be prepared by simply mixing the lipid-modified aptamer and nucleic acid motif, preferably in a 1:1 ratio, with the drug. Preferably the drug is used in excess, for example at 10-fold excess. The forming of the hybrid nanoconstruct may be followed by a purification step, such as using spin filtration.

The nucleic acid-based assembly advantageously can exploit aptamer-mediated selective cell targeting, photo induced structure switching, and lipid-mediated self-assembly, and thus provide a hybrid assembly as a molecular carrier system that allows selective transport of intercalated cytotoxic drugs to target cells and release of the payload under light irradiation. This design offers the possibility to self-assemble multiple functional domains at once into a single nanoconstruct, such as the targeting ability of an anti-cMet aptamer, and an intercalated drug-carrying motif, compared to the limited possibility of introducing multiple functionalities into a single modified aptamer system through inherent synthetic efforts. Moreover, multiple aptamer motifs that target different biomarkers on the cell surface may be assembled in a single nanoparticle by mixing the respective lipidated aptamers to allow for a more precise targeting.

A further aspect of the present invention relates to a nucleic acid-based assembly according to the invention for use as a medicament, particularly for use in the treatment of cancer. The lipid-functionalized nucleic acid-based assembly can encompass a cell-targeting aptamer accompanied by a photo-responsive oligonucleotide motif that can selectively target and transport high doses of pharmaceutically active molecules directly into the targeted cells and release the payload upon irradiation. The assembly is able to cover the essential prerequisites for aptamer-based targeted therapeutics, from fabrication of nanoconstructs of improved serum stability to efficient cell internalization, and light-triggered release of active therapeutics. Advantageously the stability of the nanocontruct and improved cell internalization can be provided by lipidation. Particularly using the targeting ability of an anti-cMet aptamer a selective transport into target cancer cells and a highly efficient cell-uptake of the hybrid-aptameric nanoconstruct into cancer cells could be demonstrated, as well as an improved effect on tumor cells by releasing an anti cancer drug inside the cell by a light trigger.

The nucleic acid-based assemblies are useful for a variety of cancers, but particularly are useful for solid tumors. As used herein, the term "solid tumor" refers to a solid mass of cancer cells that grow in organ systems and can occur anywhere in the body. In embodiments, the solid tumors are selected from the group comprising breast cancer, prostate cancer, colorectal cancer, ovarian cancer, thyroid cancer, lung cancer, liver cancer, pancreatic cancer, gastric cancer, melanoma (skin cancer), lymphoma and glioma.

For use as a medicament the nucleic acid-based assembly can be used or included in a composition. Accordingly, in another aspect the present invention relates to a pharmaceutical composition comprising as an active ingredient a nucleic acid-based assembly according to the invention. The pharmaceutical composition particularly is suitable for use in the treatment of cancer, particularly in the treatment of solid tumors. The nucleic acid-based assembly can be dissolved or dispersed in a pharmaceutically acceptable carrier. The term "pharmaceutical or pharmacologically acceptable" refers to molecular entities and compositions that do not produce an adverse, allergic or other untoward reaction when administered to a subject, such as, for example, a human, as appropriate. The pharmaceutical carrier can be, for example, a solid, liquid, or gas. Suitable carriers and adjuvants can be solid or liquid and correspond to the substances ordinarily employed in formulation technology for pharmaceutical formulations. For compositions convenient pharmaceutical media may be employed. For example, water, buffers, and the like may be used to form liquid preparations such as solutions.

The present invention also relates to the use of a nucleic acid-based assembly according to the invention for the manufacture of a medicament, preferably for the manufacture of a medicament for the treatment of cancer, particularly solid tumors.

A further aspect of the present invention relates to a method of treating cancer, particularly solid tumors, the method comprising the step of administering to a subject a therapeutically effective amount of a nucleic acid-based assembly according to the invention. Subjects include both human subjects and animal subjects, particularly mammalian subjects such as human subjects or mice or rats for medical purposes. The term "therapeutically effective amount" is used herein to mean an amount or dose sufficient to cause a therapeutic benefit such as an improvement in a clinically significant condition in the subject.

Particularly cancer refers to solid tumors. Solid tumors may be selected from the group comprising breast cancer, prostate cancer, colorectal cancer, ovarian cancer, thyroid cancer, lung cancer, liver cancer, pancreatic cancer, gastric cancer, melanoma (skin cancer), lymphoma and glioma.

Unless otherwise defined, the technical and scientific terms used herein have the same meaning as commonly understood by one of ordinary skill in the art to which this invention belongs.

The examples that follow serve to illustrate the invention in more detail but do not constitute a limitation thereof.

The figures show:
- Figure 1: A schematic representation of a lipid-modified nucleic acid motif. X represents the DMAB moiety.
- Figure 2: A schematic representation of the DMAB moiety in trans position and in UV induced cis position.
- Figure 3: A schematic representation of a nucleic acid-based assembly according to an embodiment of the invention.
- Figure 4: A maximum fluorescence intensities at λ = 669 nm (I₆₆₉) as a function of increasing concentration of Atto550 labeled nucleic acid motif.
- Figure 5: The size distribution of the heterogeneous assembly of lipid-modified aptamer and nucleic acid motif in a 1:1 ratio.
- Figure 6: in figure 6a) the growth inhibition (MTT) assays at different cell densities and in figure 6b) the average relative cell viabilities for treated H1838 cells (error bars: n = 2 ± SD).

### Materials:

All chemicals including doxorubicin (DxR) were purchased from Sigma-Aldrich unless otherwise specified and were used as received. cMet-Fc, which represents the ectodomain of cMet fused to the Fc domain of human IgG1 was purchased from R&D Systems. Wheat Germ Agglutinin, Alexa Fluor® 488 Conjugate and Hoechst 33342 were purchased from Life Technologies (Grand Island, NY, USA). γ-³²P labeled ATP (250 µCi) was purchased from PerkinElmer Health Science B. V., The Netherlands. T4 Polynucleotide kinase and 1x Polynucleotide buffer were obtained from New England Biolabs, Frankfurt a. M., Germany. Binding buffer used for the aptamer competition-binding assay was prepared by adding *E.coli* tRNA (Roche AG, Mannheim, Germany), BSA (Thermo Fischer Scientific) into the Dulbecco's PBS (Gibco, Life Technologies).

All solvents, reagents and building blocks for oligonucleotide synthesis were obtained from Proligo, Hamburg, Germany.

### Methods:

### Cell culture:

The human NSCLC cell line H1838 was obtained from the American Type Culture Collection (ATCC) and grown in T-75 cm² flasks using Dulbeccos RPMI 1640 (Invitrogen) supplemented with 10% fetal calf serum (FCS) in a humidified atmosphere at 37 °C and 5% CO₂. Cell lines were subculture twice a week at a ratio of 1:4 depending on the confluence and cell density was determined with hemocytometer before each experiment. Cells were detached using 1 ml Trypsin-EDTA solution (Sigma-Aldrich) followed by neutralization with 25 ml of RPMI media and the cells were collected by centrifugation for 5 min at 400 rpm.

### Confocal microscopy:

In vitro cell imaging of the cell internalization studies were performed using fluorescence microscopy. Prior to each experiment one 70 to 80% confluent flask was trypsinised and suspended with 10 ml of cell medium. 10 µL of the cell suspension was pipetted onto a hemocytometer and the cells were counted. Twenty-four hours prior to the internalization experiments approximately 10,000 NSCLC cells were seeded in 96-well glass bottom multiwell cell culture plates (MatTek^{®} Corporation). The plates were then incubated for 24 hours at 37°C in 5% CO₂-atmosphere. After 24 hours of incubation the cells were first washed with 1x PBS buffer and incubated with various labeled lipid-modified aptameric constructs (trCLN3-L4, trCLN3.mut-L4), the assembly (HyApNc-DxR) or free doxorubicin, in 100 µL of RPMI 1640 with 10% FCS medium containing 1 mM MgCl₂ at 37°C and 4 °C separately for 2 hours. The final concentrations of the labeled micelles were fixed at 10 µM.

Afterwards, cells were washed with fresh medium and Dulbeccos 1x PBS followed by 10 min fixation with 200 µL of a 3.7% (w/v) paraformaldehyde solution in Dulbeccos 1x PBS. Fixed cells were washed with fresh medium and Dulbeccos 1x PBS followed by staining with 200 µL of nuclear and plasma membrane staining reagent [60 µL (1mg/ml) of Alexa Fluor 488-WGA and 20 µL of Hoechst 33342 (1 mM) in 4.0 mL in 1x PBS buffer] and incubated for 10 minutes at 37 °C. After 10 minutes, the labeling solutions were removed and the stained cells were washed with 1x PBS (2x 200 µL) followed by addition of 200 µL of 1x PBS buffer.

Finally the 96 well plate was mounted with a multi-well plate holder and the confocal imaging of the fixed cells was performed by using a NikonTi-E Eclipse inverted confocal laser-scanning microscope equipped with a 60x Plan Apo VC Oil-immersion DIC N2 objective, a Nikon C2 plus confocal-laser scan head and a pinhole of 1.2 airy unit (30 µm).

The laser scanning Nikon Confocal Workstation with Galvano scanner, and lasers 408, 488, 561 and 637 nm was used, attached to a Nikon Eclipse Ti inverted microscope. Images were captured in 1024x1024 pixels format using NIS-Elements software (Nikon Corporation) and the raw images were processed using ImageJ software. The standardized optical setups of imaging, pin-holes, objective, laser power and photomultiplier gain were kept constant while recording the data for all measurements,

### Atomic force microscopy (AFM):

All AFM images of the lipid-modified aptamer and assembly aggregates were taken by using a Nanowizard III AFM (JPK instruments, Berlin) in tapping mode. ACTA probes with silicon tips were used for imaging in dry mode in air. A volume of 3µL (5 mM) of a solution of magnesium acetate (MgAc₂) in water was deposited on a freshly cleaved mica surface layer and allowed to incubate for 3 minutes and afterwards the surface was rinsed with 2x 10 µL of milli-Q water and dried under air pressure. For imaging a volume of 3µL of the lipid-modified aptamer and assembly solutions in ultra pure water were spotted on the pre-treated mica surface and allowed to incubate for 1 min. After an incubation time of 1 min on the mica surface the excess sample solution was gently shaken off and the mica surface was blown dry with air pressure and mounted to the AFM microscope for immediate imaging. The raw AFM data were processed using JPK processing software.

### ESI mass spectrometry:

Molecular weights of the lipid-modified aptamer and nucleic acid motif motifs were analyzed by ESI-LCMS in negative ion mode using a Bruker Esquire 6,000 ion-trap MS system with an electrospray ionization source coupled to an Agilent 1100 series HPLC system with a ZORBAX SB-18 analytical column (2.1x50mm). An elution buffer (10mM TEA + 100mM HFIP) in combination with linear gradients of acetonitrile from 0% to 80% in 30 minutes was used as mobile phase for analysis. The m/z ratio was calculated by deconvolution of the ionic fragments.

### Example 1

### Manufacture of a lipid-modified cMet targeting aptamer

The *anti*-cMet aptamer (trCLN3) and its lipid-modified derivatives were synthesized according to the phosphoramidite protocol using an ABI 3400 synthesizer (Applied Biosystems).

### 1.1 Synthesis of lipid-modified 5'-DMT-2'-deoxyuridine-phosphoramidite

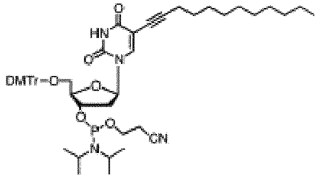

5-(1-Dodecynyl)-modified 5'-DMT-2'-deoxyuridine-phosphoramidite with the C₁₂-lipid chain incorporated at the 5-position of uridine-phosphoramidite was synthesized from 5-Iodo-2'-deoxyuridine as starting material using 1-dodecyne as starting material for the lipid, in the presence of Cu (I) iodide and Pd(Ph₃P)₄ as described in the Supporting Information of Kwak et al. J. Am. Chem. Soc. 2010, 132: 7834-7835.

### 1.2 Manufacture of a lipid-modified cMet targeting aptamer

Four of the C₁₂-lipid modified bases of step 1.1 were attached to the 5'-end of the anti-cMet aptamer trCLN3, the 40 nucleotide DNA oligonucleotide of SEQ ID NO: 1, thereby introducing four lipid-modifications to each aptamer.

The manufacture was performed in a single process using a standard phosphoramidite solid-phase DNA synthesis protocol in a scale of 200 nmol using an ABI 3400 DNA synthesizer. The lipid-modified uridine-phosphoramidite of step 1.1 (0.221 g) was dissolved in DNA-grade dichloromethane (2.7 mL) under argon atmosphere to give a 0.1 M solution.

After the last detritylation step the lipidated-uridine phosphoramidite was coupled to the detritylated 5'-end of the oligonucleotide chain. Subsequently, deprotection of phosphate groups and protected amino nucleobases as well as cleavage of the product from the solid support was carried out by incubation in a 50:50 (v/v) mixture of 30% ammonia solution (400 µl) and methyl amine (400 µl) for 2 h at 55 °C. The solid support was then removed by filtering and was washed with an ethanol/water (50:50, v/v) mixture. The filtrate was concentrated under reduced pressure and dried.

Following deprotection and removal from the solid-support, the lipid-functionalized aptamer was purified by using reversed-phase high performance liquid chromatography on an Eclipse XBD C18 column using 0.1 M TEAAc (A) and acetonitrile (B) with a gradient of A/B = 98/2 -> 35/65 in 30 minutes. The coupling yield of the labeling reaction was determined to be 31 % by integration of the peaks in the HPLC chromatogram. The purified lipid-modified oligonucleotide fraction in TEAAc buffer was concentrated using a freeze-dryer. Oligonucleotide concentrations were determined by UV absorbance using extinction coefficients at λ = 260 nm. Finally, the identity of the oligonucleotides was confirmed by ESI-mass spectrometry using a Bruker Esquire 6,000 iontrap MS system.

The resulting lipid-modified aptamer comprising four C₁₂-lipid modified bases at the 5'-end of the anti-cMet aptamer trCLN3 of SEQ ID NO: 1, which in following also is denoted "trCLN3-L4".

1.3 Determination of the effect of lipid-modifications on cMet binding To test the effect of the lipid-modification on binding properties, IC₅₀ values were determined by a competitive filter retention assay in which varying concentrations of unlabeled 5'-(1-dodecynyl)-functionalized aptamer competed with constant amounts of γ-³²P-labeled aptamer trCLN3 of SEQ ID NO: 1 in binding to cMet. Two control experiments were also performed using unlabeled trCLN3 aptamer and its non-binding two point mutant variant 5'-TGGATGATAGCTCGGTCGGGGTGGATGGGTTGGCAAGTCT-3' (SEQ ID NO: 3) as competitors.

First, the aptamer trCLN3 of SEQ ID NO: 1 was 5'-end-labeled with γ-³²P-ATP using T4 polynucleotide kinase. An aliquot of 20 µL solution containing 50 pmol trCLN3, 6.7 pmol γ-³²P-ATP and 20 U T4 polynucleotide kinase in 1x polynucleotide kinase buffer (New England Biolabs) was incubated at 37 °C for 45 min, followed by removal of unreacted γ-³²P-ATP using an Illustra G-25 microspin column (GE Healthcare, München, Germany). The purity of the radiolabeled aptamer was confirmed using a 10% PAGE-gel.

To determine the affinity, ∼25 fmol of radiolabeled aptamer was incubated with a cMet concentration of ∼50 nM together with varying concentrations (1 µM - 25 pM) of unlabeled competitor for 30 min at 37 °C in 25 µL of buffer containing 0.1 mg/ml *E.coli* tRNA (Roche, Mannheim, Germany), 0.25 mg/ml BSA, 2 mM MgCl₂ in 1x PBS, pH 7.4. The aptamer protein complexes were captured on a Protran nitrocellulose membrane (GE Healthcare) that was pre-incubated in 0.4 M KOH for 10 minutes, followed by washing with 1x PBS containing 2 mM MgCl₂, pH 7.4. After addition of the aptamer-protein solution, the filter was washed 4 times with 1x PBS containing 2 mM MgCl₂ using vacuum filtration. Residual radioactivity due to cMet bound labeled aptamers was quantified using Fujifilm Fla-3000 PhosphorImager and AIDA software. The curves were fitted with GraphPadPrism 3.02 plotting non-linear regression curve and the IC₅₀ values have been calculated assuming a competition for single binding site.

Strong cMet binding was observed for the lipid-modified aptamer trCLN3-L4 with an IC₅₀ value of 43 nM, compared to 56 nM obtained for the non lipid-modified native aptamer trCLN3. This result demonstrates that the lipid-modified aptamer retained the binding affinity to cMet as compared to the non-modified aptamer trCLN3. In contrast, the lipid-modified mutant aptamer could not compete with the ³²P-trCLN3 for binding to cMet within the tested concentration range, indicating that the displacement of the non-lipidated ³²P-trCLN3 from its bound cMet-target by its lipidated counterpart trCLN3-L4 is specific.

### 1.4 Determination of the effect of lipid-modification on serum nuclease stability

Since the application of the aptamer as an effective therapeutic tool depends on its half-life, serum stabilities of the native aptamer (trCLN3) and the lipid-modified aptamer (trCLN3-L4) was investigated in fetal calf serum (FCS).

For this purpose, the aptamer motifs have been labeled at their 5'-end with ³²P to form radiolabeled oligonucleotides. The degradation test of all the aptamer motifs were performed for 60-72h at 37 °C. 6 pmol (12 µl of 0.5 µM) of radio-labeled aptamer (5'-end-labeled with γ-³²P) was incubated in a volume of 300 µl freshly thawed PBS-buffered fetal calf serum (270 µl FCS + 30 µl 10x PBS). For each measurement (10 µl of samples were removed and mixed with 90 µl of gel loading buffer (80% formamide + 5 mM EDTA + 0.01 % SDS) and subsequently stored at -20 °C. Aliquots of samples were taken after indicated time intervals of 0, 0.3, 1.5, 3, 6, 24, 48, 60 and 72 h respectively. The serum stability of the aptamer in FCS at different time intervals were analyzed on a denaturing PAGE by loading 10 µl of each sample onto a 10% TAE-Urea gel and running the gels for 90 minutes at 350 V. Gels were wrapped in clingfilm and exposed to a phosphorimager screen in a closed cassette over a period of 12h and finally the residual intact aptamer bands were analyzed by scanning the screen in a phosphorimage-scanner (FujiFilm FLA 3000). Intensities of the residual intact aptamer bands were calculated applying AIDA image analyzer software program. Serums half-lives of the selected aptamers were determined by using a half-life curve-fitting data analysis program (GraphPad Prism). The half-life of the radiolabeled aptamer (trCLN3) was determined to be 8.7 h in freshly thawed 90% fetal calf serum, while the lipid-modified aptamer derivative (trCLN3-L4) showed no significant degradation even up to 72 h of incubation.

In a control experiment, the serum stabilities of the non-binding mutant variant of SEQ ID NO: 3 and its lipid-modified derivative were tested and determined to 2.8 h and 23.6 h, respectively.

The half-life data show that the serum stabilities of the lipid-modified aptamer are significantly higher than that of the non-lipidated variant. These observations clearly indicate that the lipid modification protects the aptamer against enzymatic degradation thereby increasing the serum stability several fold.

### 1.5 Determination of critical micelle concentration of lipid-modified aptamers

The lipid-modified aptamers showed a strong tendency to self-aggregate in aqueous solution by forming spherical nanoconstructs above a critical micelle concentration (CMC) at room temperature.

The critical micelle concentration (CMC) value of the lipid-modified aptamer aggregates was determined by intermolecular Förster resonance energy transfer (FRET) experiments using a FRET pair of 6-Fam and Atto647N both attached to the 5'-end of the lipid-modified aptamer. The FRET labels were attached at the 5'-end in immediate proximity to the lipid-modifications to ensure that intermolecular FRET effects report the formation of micellar nanoconstructs at a concentration above the critical micelle concentration. In the FRET experiment, a series of nanoconstructs was self-assembled by mixing 6-Fam- and Atto647N-labeled lipid-modified aptamer in 1:1 ratios in a concentration range between 0.035-15 µM. The solutions were incubated at 70 °C for 10 minutes in the dark and slowly cooled down to room temperature overnight at a rate of 1 °C per 10 minutes. The mixtures were transferred into a 384-well plate and the FRET effect was monitored at room temperature by using an excitation wave length of λₑₓ = 480 nm and an emission wavelength of λₑₘ = 669 nm using an EnSpire^{®} Multimode Plate Reader (PerkinElmer).

The intensity signals were collected for both FRET channels at λₑₘ = 669 nm for the acceptor channel and that of donor channel at λₑₘ = 520 nm. The concentration dependent intensity ratios (I₆₆₉/I₅₂₀) were plotted as a logarithmic function depending on the concentration of the lipid-modified aptamer. The CMC value was determined from the intersection of the lower horizontal asymptote of the sigmoidal curve with the tangent at the inflection point corresponding to the minimum concentration required for formation of stable micelles in aqueous medium. The CMCs of trCLN3-L4 aggregate was determined to be 300 nM (∼ 0.005 mg/ml).

Critical micelle concentration (CMC) value of the lipid-modified aptamer was further confirmed by internalizing pyrene into the hydrophobic-lipid core of the micellar aggregate followed by measuring the fluorescence of pyrene-loaded lipid-modified aptamer nanoconstructs at different concentrations. For this experiment a fixed amount of pyrene in acetone was transferred to an empty tube and acetone was allowed to evaporate in the dark at 45 °C for 30 min using an Eppendorf concentrator. Solutions of lipid-modified aptamer in the concentration range between 0.0005-0.5 mg/ml was then added to yield a final pyrene concentration fixed at 100 µM for all reactions. The solutions were incubated at 90 °C for 10 minutes in the dark and slowly cooled down to room temperature overnight at a rate of 1°C / 10 min in order to internalize pyrene into the hydrophobic lipid core. The pyrene-loaded lipid-modified aptamer nanoconstructs were transferred into a 384-multi well plate and the fluorescence emission spectrum of each well was recorded at room temperature by using an excitation wave length of 339 nm in an EnSpire^{®} Multimode Plate Reader (PerkinElmer).

In close proximity, two pyrene molecules form an excimer that emits fluorescence at a longer wavelength compared to the monomer emission. The formed excimer is a dimeric complex where one molecule exists in an excited state and the other molecule in a ground state. Monomer emission of pyrene occurs within a range of 360-400 nm whereas the excimer emission is obtained within the wavelength limit of 465-500 nm. The critical micelle concentration was determined by the distinguishable pyrene excimer fluorescence of the corresponding DNA concentration

Both methods thus consistently yielded CMC values in the range of 300-350 nM concentrations.

### 1.6 Determination of the size of the micelles of lipid-modified aptamer

The size and morphology of the micelles of of lipid-modified aptamer were studied by atomic force microscopy (AFM) as described above and electron microscopy (TEM). To obtain a statistical evaluation of the size-distribution of nanoconstructs, the diameters of at least 50 nanoconstructs for each AFM image were compiled in histograms and fitted by Gaussian distributions. It was observed that the micelles of of lipid-modified aptamer showed an average size of 21.2 ±1.5 nm. This was consistent with a size of 25 nm measured by TEM.

### Example 2

### Manufacture of a lipid-modified nucleic acid motif intercalating doxorubicin

2.1 Synthesis of DMT-protected 2',6'-dimethylazobenzene phosphoramidite DMT-protected phosphoramidite carrying a 2',6'-dimethylazobenzene moiety on a D-threoninol backbone was synthesized as described in H. Asanuma et al., Nature Protocols 2007, 203-212.

### 2.2 Manufacture of a lipid-modified nucleic acid motif

The nucleic acid motif containing four 2',6'-dimethylazobenzene-D-threoninol residues of SEQ ID NO: 2 (5'-GCNGCGNCTCNGCGNCGATTATTACGCGCGAGCGCGC-3' wherein N is a 2',6'-dimethylazobenzene-D-threoninol residue) was synthesized by solid phase DNA-synthesis and four 5-(1-Dodecynyl)-modified 5'-DMT-2'-deoxyuridine bases were introduced at the 5'-end as described in example 1.2 and purified. The molecular mass of the lipid-modified nucleic acid motif was analyzed by ESI-LCMS in negative ion mode (Bruker Esquire 6,000 ion-trap MS system with an electrospray ionization source coupled to an Agilent 1100 series.). Deconvolution of the ionic fragments of the ESI mass spectrum of the purified lipid-modified nucleic acid motif lead to a measured total mass of MWₘₑₐₛ = 13564.25 corresponding to the target oligonucleotide with the calculated mass of MW_{calc} = 13563.51.

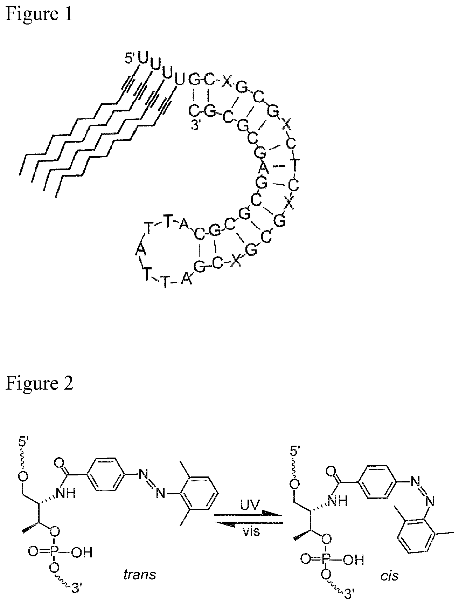

The figure 1 shows a schematic representation of the lipid-modified nucleic acid motif. The figure shows the hairpin-duplex motif with repetitive 5'-CG-3' base pairs for doxorubicin intercalation. The positions of the four 2',6'-dimethylazobenzene (DMAB) moieties on a D-threoninol backbone are marked with a cross (X). Four C₁₂-lipid modified deoxyuridine residues are attached to the 5'-end.

### 2.3 Investigation of photo-switching

The schematic of the switch mechanism mediated by of the four photo-responsible 2',6'-dimethylazobenzene (DMAB) moieties contained in the nucleic acid motif was investigated by UV/vis-spectroscopy.

UV/vis-spectra of the nucleic acid motif were taken in a range between λ = 300 and λ = 420 nm, and showed two sets of curves for a reversible photo switching of the DMAB moiety for alternating irradiation with UV and visible light. The absorption maximum was determined at λ = 345 nm. The switching process was fully reversible and could be repeated for at least 5 irradiation cycles. Probes of the nucleic acid motif were taken at the UV and vis-irradiated state upon repeated irradiation with UV- and visible light for 5 minutes each and analyzed by PAGE gel electrophoresis. The DMAB-modified GC-rich hairpin structure showed a change in electrophoretic shift, which is consistent with significant structural changes between hairpin and unpaired motif.

The figure 2 shows a schematic representation of the cis and trans-conformation of the 2',6'-dimethylazobenzene (DMAB) groups.

### 2.4 Intercalation of doxorubicin into the nucleic acid motif

The intercalating of multiple doxorubicin molecules per each nucleic acid motif was investigated by binding studies between the lipid-modified nucleic acid motif and doxorubicin. A fixed concentration of 10 µM of doxorubicin was incubated with an increasing molar ratio of 1 - 7 µM (0.1-0.7 equiv.) of the lipid-modified nucleic acid motif in PBS solution. Fluorescence emission spectra (λₑₓ = 480 nm) were taken between 500 nm and 750 nm, and showed a gradual reduction in the fluorescence intensity of doxorubicin with an increasing concentration of added nucleic acid motif.

To test the difference in binding affinity of the nucleic acid motif for cis and trans-conformation of the DMAB groups, the nucleic acid motif was separately irradiated with visible light (λ = 450 nm) and UV light (λ = 365 nm) for 5 minutes each and mixed with a fixed concentration of 10 µM doxorubicin while the concentration of the nucleic acid motif was varied from 0.1-0.7 equivalents to that of the doxorubicin concentration. The resulting fluorescence curve of the nucleic acid motif with DMAB in trans-conformation (λ = 450 nm) showed a higher reduction in fluorescence intensity with an increasing molar equivalent of added nucleic acid motif as compared to the nucleic acid motif in which the DMAB moieties were in cis-conformation. The difference in fluorescence intensity was about 30% higher in case of trans-DMAB than in cis-DMAB.

This difference in fluorescence intensities further supports the conclusion, that the DMAB modified nucleic acid motif is opened by irradiation with UV-light thereby releasing doxorubicin.

### Example 3

### Manufacture of a nucleic acid-based assembly

### 3.1 Assembly of the lipid-modified aptamer and the lipid-modified nucleic acid motif without drug

The fabrication of the assembly was performed by employing a simple heating and cooling procedure. An aqueous solution of 250 pmol of the lipid-modified aptamer of example 1 was added to 250 pmol of the lipid-modified nucleic acid motif of example 2.2 dissolved in a volume of 50 µl milli-Q H₂O (10 µM solution). The resulting solution was heated to 90 °C, the temperature was kept for 10 minutes and subsequently cooled down to a temperature of 10 °C at a rate of 1 °C /10 minutes time interval. Hybrid micellar nanoconstructs formed in the aqueous solution, induced by micro phase separation.

### 3.2 Assembly of the lipid-modified aptamer and the lipid-modified nucleic acid motif with doxorubicin

The nucleic acid-based assembly carrying a drug was prepared by mixing the lipid-modified aptamer of example 1 and the lipid-modified nucleic acid motif of example 2.2 in 1:1 ratio with a 10-fold excess of Doxorubicin in binding buffer (1x PBS + 5 mM EDTA + 0.01 % SDS). The solution was incubated at 90 °C for 10 minutes and slowly cooled down to room temperature overnight at a rate of 1 °C / 10 min in order to intercalate doxorubicin into the nucleic acid motif.

The drug-loaded assembly was transferred to an Amicon^{®}Ultra-0.5 centrifugal filter column with 3K molecular weight cutoff and excess of free doxorubicin which is not intercalated into the nucleic acid motif was removed by three times consecutive centrifugation at 14,000x g for 10 minutes at room temperature while adding fresh binding buffer at each centrifugation step. After each centrifugation step, a UV/Vis- spectrum of the flow through washing was recorded and a reduction in doxorubicin absorbance further confirmed the successive removal of excess doxorubicin through repeated washing.

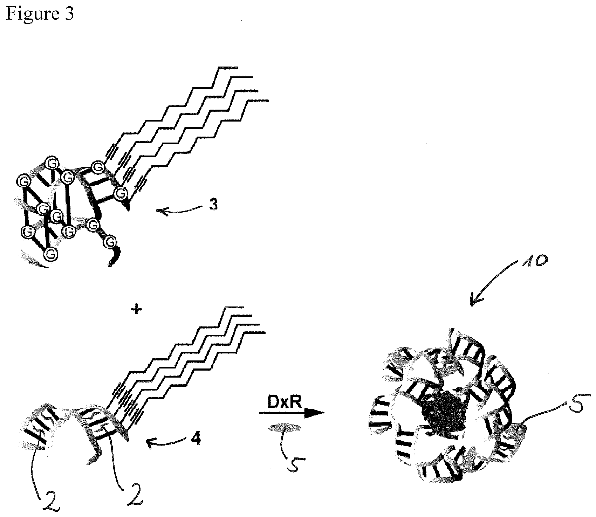

The figure 3 shows a schematic representation of the nucleic acid-based assembly 10, comprising lipid-modified nucleic acid aptamers 3 and the lipid-modified nucleic acid motifs 4, which comprises 2',6'-dimethylazobenzene (DMAB) groups 2 as photo-responsive moieties. The nucleic acid motifs 4 intercalate the drug doxorubicin 5 (DxR). The lipid-modified aptamers 3 and nucleic acid motifs 4 form the assembly 10 through noncovalent interaction and the assembly comprises an outer shell of DNA formed by the nucleic acids of aptamer and the nucleic acid motif, and an inner core of hydrophobic lipids.

### Example 4

### Determination of lipid-mediated self-assembly

To determine whether the lipid-modified nucleic acid motif and the lipid-modified aptamer form an assembly through noncovalent interaction, lipid-mediated self-assembly was monitored via intermolecular Förster resonance energy transfer (FRET) experiments with dye-labeled aptamer and nucleic acid motif.

The dyes Atto-647N and Atto550 were both attached to the 5'-end of the lipid-modified aptamer and nucleic acid motif of example 2, respectively. The Atto dye labels were attached at the 5'-end in immediate proximity to the lipid-modifications to ensure that intermolecular FRET effects report the formation of micellar nanoconstructs. Atto-647N-labeled aptamer and Atto-550-labeled nucleic acid motif were mixed in different ratios. For this 5 µM Atto-647N-labeled aptamer and Atto-550-labeled nucleic acid motif were mixed in a concentration range between 1 µM and 15 µM. The intensity signals were collected for both FRET channels at λₑₘ = 669 nm for the acceptor channel and that of donor channel at λₑₘ = 576 nm. The concentration dependent intensity ratios (I₆₆₉/I₅₇₆) were plotted as a logarithmic function depending on the concentration of the lipid-modified aptamer. The following table 1 summarizes the concentrations and intensity ratios:

**Table 1: Concentrations [µM] of Atto-labeled motif and aptamer mixed in different ratios to form hybrid micellar nanoconstructs**

| Exp. No. | Atto550-labeled motif [µM] | Atto647N-labeled aptamer [µM] | volume (µL) | equivalents Atto550 labeled motif | I₆₆₉^{a} [mean ± sd] | I₅₇₆^{b} [mean ± sd] | I₆₆₉/I₅₇₆^{c} |
|---|---|---|---|---|---|---|---|
| 1 | 0.0 | 5.0 | 20 | 0.0 | 652 ± 206 | 41 ± 7 | 15.90 |
| 2 | 1.0 | 5.0 | 20 | 0.2 | 2317 ± 657 | 416 ± 116 | 5.56 |
| 3 | 1.75 | 5.0 | 20 | 0.35 | 5673 ± 881 | 775 ± 169 | 7.32 |
| 4 | 2.5 | 5.0 | 20 | 0.5 | 9604 ± 1172 | 1218 ± 234 | 7.88 |
| 5 | 5.0 | 5.0 | 20 | 1.0 | 21098 ± 402 | 3553 ± 434 | 5.93 |
| 6 | 7.5 | 5.0 | 20 | 1.5 | 28225 ± 1164 | 6106 ± 378 | 4.62 |
| 7 | 10 | 5.0 | 20 | 2.0 | 34010 ± 3593 | 9992 ± 153 | 3.40 |
| 8 | 15 | 5.0 | 20 | 3.0 | 35242 ± 5951 | 27766 ± 4606 | 1.26 |

| | | | | | | | |
|---|---|---|---|---|---|---|---|
| ^{a}Fluorescence intensities at λ = 669 nm. ^{b}Fluorescence intensities at λ = 576 nm. ^{c}Estimated ratio (I₆₆₉/I₅₇₆) from the FRET experiments. | | | | | | | |

The fluorescence at λₑₓ = 535 nm showed that assemblies formed with 0.2 equivalents of Atto550 labeled nucleic acid motif, i.e. a ratio of aptamer to motif of 5 : 1, yielded an intensity ratio I₆₆₉/I₅₇₆ of 5.56. In contrast, assemblies formed with 0.35 or 0.5 excess equivalents of Atto550 labeled nucleic acid motif showed an increasing I₆₆₉/I₅₇₆ value of 7.32 and 7.88, respectively. This demonstrates a significant enhancement of about 32% and about 41 % relative to the Atto647N fluorescence.

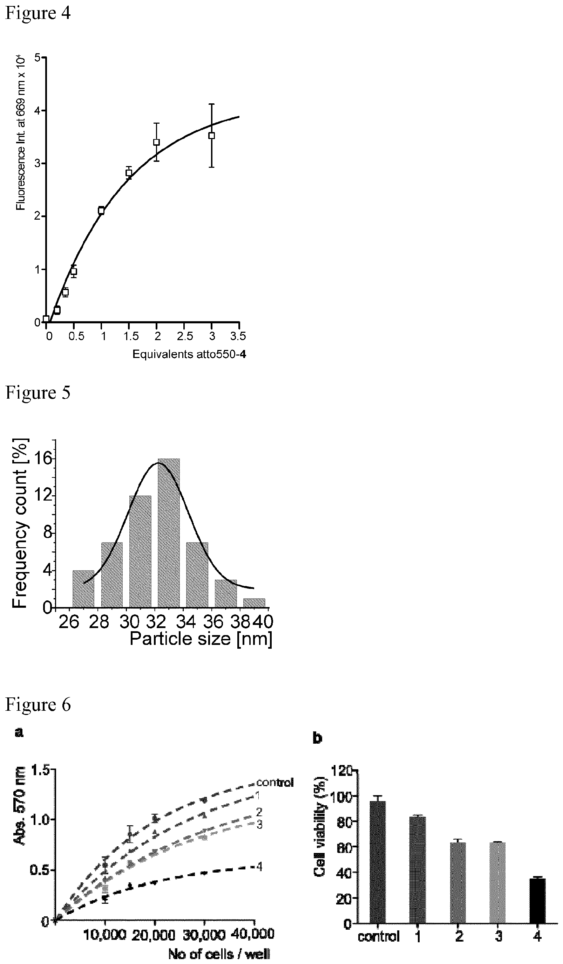

The figure 4 shows the maximum fluorescence intensities at λ = 669 nm (I₆₆₉) as a function of increasing concentration of the Atto550 labeled nucleic acid motif, denoted "atto500-4". As can be taken from figure 4, an increase in FRET observed with increasing concentrations of Atto550-4 reached saturation between 2.0 and 2.5 equivalents. Nevertheless the I₆₆₉/I₅₇₆ value already reached 5.93 at one equivalent of the Atto550 labeled nucleic acid motif (ratio = 1:1). This finding indicates that a ratio of lipid-modified aptamer and nucleic acid motif of about 1:2 to 3:2 in the assembly shows good results.

In a control experiment, the nucleic acid motif without lipid-modification was labeled with Atto550 and mixed with the Atto-647N-labeled aptamer. In this experiment, with a 1:1 ratio of lipid-modified aptamer and lipid-devoid nucleic acid motif an I₆₆₉/I₅₇₆ value of 0.09 was observed, indicating that only diffusion-controlled encounters occurred, but no stable hybrid assembly was formed.

These data provide evidence that the lipid-modified nucleic acid motif and aptamer form an assembly through noncovalent interaction in different ratios, which were stabilized by hydrophobic interaction of the lipid modification.

### Example 5

### Characterization of size and structural features of the assembly

The size and morphology of the assembly of lipid-modified aptamer and nucleic acid motif in a 1:1 ratio were further studied by atomic force microscopy (AFM) as described above. To obtain a statistical evaluation of the size-distribution of nanoconstructs, the diameters of at least 50 nanoconstructs for each AFM image were compiled in histograms and fitted by Gaussian distributions. The figure 5 shows the size distribution of the heterogeneous assembly of lipid-modified aptamer and nucleic acid motif in a 1:1 ratio. It was observed that the assembly exhibited a spherical shape of an average diameter of 32.3 ± 2.1 nm. The increase in size of the heterogenous assembly as compared to the homogenous nanoconstructs of lipid-modified aptamer may result from differences in the physico-chemical properties due to the aptamer or from structural differences, or both.

Together, the data of examples 4 and 5 provide evidence that the lipid-modified nucleic acid motif and aptamer self-assemble to form hybrid heterogeneous nanoconstructs of spherical geometry when the lipid modifications are present.

### Example 6

### In vitro analysis of cMet-mediated cellular uptake of the assembly

After confirming the sucessful fomation of the assembly, the cell targeting ability of the trCLN3 aptamer mediated by cMet recognition was investigated. Cell uptake experiments were performed with the NCI-H1838 lung cancer cell line that expresses high levels of cMet.

6.1 Determination of the uptake of lipid-modified trCLN3 aptamer into cancer cells H1838 cells were incubated with Atto647N-labeled lipid-modified trCLN3 aptamer, which forms a homogeneous micellar structure, at 37 °C for 90 min and examined by confocal microscopy as described above. A strong intracellular red-fluorescence was detected. This result indicates a highly efficient internalization of the lipid-modified trCLN3 aptamer.

The incubation was repeated at 4°C, and at this temperature showed only a weak membrane-localized fluorescence with markedly reduced Atto647-fluorescence in the cytoplasm. This finding is consistent with inhibition of endocytosis at low temperature.

### 6.2 Determination of the uptake of the assembly into cancer cells

Cellular uptake studies of an assembly comprising a mixture of lipid-modified Atto550-labeled nucleic acid motif and Atto647N-labeled aptamer in a 1:1 ratio were performed. Both fluorescent probes constitute a suitable FRET pair entrapped within the lipid core that can be employed to validate whether the functional nanoconstructs enter and target H1838 cells. The confocal images showed not only a cytosolic staining for both dyes, but also a FRET signal was observed. During confocal imaging all settings were kept constant, as in detail is described in the method section above. To evaluate the occurrence of FRET, the images were analyzed using a method where the PixFRET plugin of the image processing software ImageJ was used for FRET quantification. Briefly, the bleed-through of the acceptor and donor channels was determined and finally the calculated FRET images were reconstructed. The calculated FRET images suggested that donor and acceptor dyes are in correct geometry, supporting the integrity of the nanoconstructs. High FRET efficiencies were only observed when the assembly was able to enter the cancer cells. In control experiments, mutated nanoconstructs containing the non-cMet-binding Atto647N-labeled mutant trCLN3-L4 motif (SEQ ID NO: 3) and Atto550-labeled nucleic acid motif resulted in poor FRET efficiencies, similar to background signals.

These findings indicate that that the process of internalization is target-specific rather than occurring randomly. These results verifies that the assembly comprising a cMet-targeting aptamer successfully targets the cancer cells.

### Comparative Example 7

### In vitro analysis of cMet-mediated cellular uptake of aptamer without lipid-modification

H1838 cells were incubated with Atto647N-labeled trCLN3 aptamer at 37 °C for 90 min and examined by confocal microscopy as described in example 6. The cells showed only marginal cellular uptake of the trCLN3 aptamer without lipid-modification. This finding suggests that the lipid-modification is required for efficient uptake.

### Example 8

### Determination of the photo-triggered release of doxorubicin from the assembly

The selective transport of doxorubicin into the cells, followed by its light triggered release from the assembly was investigated. The doxorubicin-loaded assembly was prepared by mixing lipid-modified aptamer (trCLN3) and lipid-modified nucleic acid motif (1:1 ratio) with 10-fold excess of doxorubicin followed by a purification step using spin filtration, as is described in example 3.2.

To ensure minimum cell mortality upon UV-irradiation, H1838 cells were irradiated at t = 0, 5, 10, 15 and 30 minutes, respectively, at an intensity of 350 mW/cm². Cell viability as a function of time dependent response to UV treatment was measured by an MTT assay 24 h after irradiation. A maximum survival rate comparable to the non-irradiated control (t = 0 min) was observed at an irradiation time t ≤ 5 min.

To verify the ability of the assembly to selectively transport doxorubicin to target cells and to study the effect of light-triggered release of the intercalated doxorubicin, the fluorescence signal of doxorubicin in the cell nuclei and cytoplasm of H1838 cells that were incubated with free doxorubicin (as control) and the doxorubicin-loaded assembly was monitored. The release of doxorubicin from the assembly was investigated by confocal microscopy with and without subsequent irradiation at 365 nm while maintaining the concentration of doxorubicin in the assembly and free doxorubicin at 40 µM. Confocal images of the H1838 cells at 37 °C after 2 h of incubation showed a decrease in the fluorescence signal of doxorubicin observed in the cell nuclei in the following order: free doxorubicin, doxorubicin-loaded assembly with and without UV irradiation (λ=365 nm, 350 mW/cm²).

Strong doxorubicin fluorescence signals were observed from the cell nuclei treated with free doxorubicin indicating that free doxorubicin accumulates almost exclusively in the nuclear region. However, the doxorubicin-loaded assembly without light irradiation led to a considerably weaker doxorubicin-fluorescence in the nucleus and a noticeable fluorescence in the cytoplasm confirming that most of the doxorubicin is predominantly localized in the cytoplasm bound to the assembly. In contrast, when the doxorubicin-loaded assembly was exposed to irradiation at 365 nm (350 mW/cm²) a discernible increase in both nuclear and cytoplasmic fluorescence was detected.

This result indicated that after UV irradiation, most of the intercalated doxorubicin was released from the assembly and subsequently transferred into the cell nuclei. This finding verified that doxorubicin can be selectively transported into the target cancer cells by the nucleic acid-based assembly.

### Example 9

### Determination of the cytotoxicity of the doxorubicin-loaded assembly in cancer cells

The *in vitro* cytotoxicity of doxorubicin-loaded assembly and of a non-targeted assembly comprising the mutant aptamer of SEQ ID NO:3 were analyzed and compared to that of cells treated with only RPMI medium and not exposed to UV irradiation by the MTT assay.

H1838 lung cancer cells were treated with unloaded assembly (1) doxorubicin-loaded assembly (4), and assembly comprising the mutant aptamer (2) for 2h at 37 °C while maintaining the concentration of doxorubicin in the assembly and the assembly comprising mutant aptamer at 40 µM. After 2 hours of incubation, the cell medium was replaced with fresh RPMI medium and the cells were subsequently exposed to UV light for 5 minutes (λ = 365 nm; 350 mW/cm²). Afterwards the cells were further allowed to incubate for 6 h at 37 °C, before being subjected to the MTT assay. Untreated cells and cells treated with the doxorubicin-loaded assembly but without UV-irradiation (3) were used as control.

The figure 6 shows in figure 6a) the growth inhibition (MTT) assays at different cell densities and in figure 6b) the average relative cell viabilities for H1838 cells treated with un-loaded assembly and UV radiation (bar denoted 1), with doxorubicin-loaded assembly comprising the mutant aptamer and UV radiation (bar 2), and treated with the doxorubicin-loaded assembly and UV radiation (bar 4), compared to untreated cells "control" and the further control cells treated with doxorubicin-loaded assembly but without subsequent UV irradiation (bar 3).

As can be seen in the figure 6, no toxicity effects were detected for cells treated with unloaded assembly with exposure to UV light, indicating a cell survival rate comparable to non-irradiated cells treated with only RPMI medium. Cells treated with doxorubicin-loaded assembly and exposed to UV irradiation exhibited about 63% growth inhibition (bar 4) compared to the untreated control. In contrast, cells incubated with the non-targeted assembly with subsequent UV irradiation only showed about 33% growth inhibition (bar 2) compared to the untreated cells, consistent with their lower level of cellular uptake.

The 30% increase in cell mortality observed for cells treated with doxorubicin-loaded assembly compared to cells treated with the mutant, non-targeted doxorubicin-loaded assembly under the same irradiation conditions (bar 4 vs bar 2) suggest that the cMet-overexpressing H1838 cells effectively internalized the doxorubicin-loaded assembly due to receptor-mediated endocytosis, while non-targeted nanoconstructs exhibited significantly lower efficacy. This finding demonstrates a highly efficient cell-uptake into H1838 cells and an improved effect on tumor cell viability by releasing doxorubicin inside the cell by a light trigger.

In summary, the examples show that by exploiting aptamer-mediated selective cell targeting, photoinduced structure switching, and lipid-mediated self-assembly a hybrid aptamer-nanoconstruct was developed as a molecular carrier system that allows selective transport of intercalated cytotoxic drugs to target cells and release of the payload under light irradiation. Particularly it could be demonstrated that the self-aggregated nanoconstruct assemblies were stabilized in aqueous solution through hydrophobic interaction of the lipids.

The mixed nature of the nanoconstructs and their size was confirmed by FRET studies and AFM measurements. Examples 8 and 9 further demonstrated a highly efficient cell-uptake of the designed hybrid assembly into H1838 cells and an improved effect on tumor cell targeting by releasing doxorubicin inside the cell by a light trigger. Particularly it could be demonstrated that lipid-modification of the aptamer and the drug-intercalating nucleic acid motif lead to a stable nanoconstruct with high resistance against nucleases accompanied by much improved cell-uptake compared to the unmodified aptamer.

The assembly advantageously is widely applicable for the simultaneous delivery of a variety of different regulatory molecules, with high specificity and efficiency to specifically block functions of disease-relevant biomolecules.

## Claims

1. A nucleic acid-based assembly comprising:
- at least one nucleic acid aptamer, and
- at least one nucleic acid motif designed to physically capture a drug, wherein the nucleic acid motif comprises one or more photo-responsive moieties that effect the release of the drug upon irradiation,
wherein the aptamer and the nucleic acid motif each are covalently linked to one or more lipids, wherein the lipid-modified aptamer and nucleic acid motif form the assembly through noncovalent interaction.

2. The nucleic acid-based assembly according to claim 1, wherein the lipid is selected from the group comprising C₁₂, C₁₄, C₁₆ and C₁₈ saturated and unsaturated fatty acids.

3. The nucleic acid-based assembly according to claims 1 or 2, wherein the aptamer and/or the nucleic acid motif comprise a terminal lipid modification, preferably in a range from 2 to 6, more preferably 3, 4 or 5 lipids attached to the 5'-end.

4. The nucleic acid-based assembly according to any one of the foregoing claims,
wherein the aptamer targets the hepatocyte growth factor receptor, wherein the aptamer particularly comprises the sequence SEQ ID NO: 1.

5. The nucleic acid-based assembly according to any one of the foregoing claims,
wherein the nucleic acid motif is a 5'-GC rich oligodeoxynucleotide that forms one or more hairpin loops that intercalate the drug.

6. The nucleic acid-based assembly according to any one of the foregoing claims,
wherein the photo-responsible moiety is an azobenzene group, preferably 2',6'-dimethylazobenzene.

7. The nucleic acid-based assembly according to any one of the foregoing claims,
wherein the nucleic acid motif comprises the nucleotide sequence 5'-GCNGCGNCT CNGCGNCGATTATTACGCGCGAGCGCGC-3' (SEQ ID NO: 2), wherein N is a 2',6'-dimethylazobenzene-D-threoninol residue.

8. The nucleic acid-based assembly according to any one of the foregoing claims,
wherein the drug is an anti-cancer drug, preferably doxorubicin.

9. The nucleic acid-based assembly according to any one of the foregoing claims,
wherein the assembly comprises a second aptamer, preferably an aptamer targeting a cancer biomarker protein, or a protein that is expressed on a target cell.

10. The nucleic acid-based assembly according to any one of the foregoing claims,
wherein the drug is released upon irradiation by visible light, ultraviolet light, or X-ray.

11. The nucleic acid-based assembly according to any one of the foregoing claims,
wherein the lipid-modified aptamer and nucleic acid motif are present in the assembly in a ratio in a range from ≥ 1:2 to ≤ 3:2, preferably in a 1:1 ratio.

12. The nucleic acid-based assembly according to any one of the foregoing claims for use as a medicament, preferably for use in the treatment of cancer, particularly of solid tumors.

13. A pharmaceutical composition comprising as an active ingredient a nucleic acid-based assembly according to anyone of claims 1 to 12, preferably for use in the treatment of cancer, particularly for use in the treatment of solid tumors.

14. Use of a nucleic acid-based assembly according to anyone of claims 1 to 12 for the manufacture of a medicament, preferably for the manufacture of a medicament for the treatment of cancer, particularly of solid tumors.

15. A method of treating cancer, particularly solid tumors, the method comprising the step of administering to a subject a therapeutically effective amount of a nucleic acid-based assembly according to anyone of claims 1 to 12.


SEQUENCE LISTING

<110>  Rheinische Friedrich-Wilhelms Universität Bonn

<120>  Nucleic acid-based assembly and use of the assembly in cancer
       therapy

<130>  UD 40861/SAM

<160>  3

<170>  PatentIn version 3.3

<210>  1
<211>  40
<212>  DNA
<213>  Artificial

<220>
<223>  aptamer

<400>  1
tggatggtag ctcggtcggg gtgggtgggt tggcaagtct                              40


<210>  2
<211>  37
<212>  DNA
<213>  artificial

<220>
<223>  nucleic acid motif


<220>
<221>  misc_feature
<222>  (3)..(3)
<223>  N is a 2',6'-dimethylazobenzene-D-threoninol residue

<220>
<221>  misc_feature
<222>  (7)..(7)
<223>  N is a 2',6'-dimethylazobenzene-D-threoninol residue

<220>
<221>  misc_feature
<222>  (11)..(11)
<223>  N is a 2',6'-dimethylazobenzene-D-threoninol residue

<220>
<221>  misc_feature
<222>  (15)..(15)
<223>  N is a 2',6'-dimethylazobenzene-D-threoninol residue

<400>  2
gcngcgnctc ngcgncgatt attacgcgcg agcgcgc                                 37


<210>  3
<211>  40
<212>  DNA
<213>  artificial

<220>
<223>  aptamer

<400>  3
tggatgatag ctcggtcggg gtggatgggt tggcaagtct                          40

SEQUENCE LISTING

<110>  Rheinische Friedrich-Wilhelms Universität Bonn

<120>  Nucleic acid-based assembly and use of the assembly in cancer
       therapy

<130>  UD 40861/SAM

<140>  EP16202754.4
<141>  2016-12-07

<160>  4

<170>  PatentIn version 3.3

<210>  1
<211>  40
<212>  DNA
<213>  Artificial

<220>
<223>  aptamer

<400>  1
tggatggtag ctcggtcggg gtgggtgggt tggcaagtct                              40


<210>  2
<211>  37
<212>  DNA
<213>  artificial

<220>
<223>  nucleic acid motif


<220>
<221>  misc_feature
<222>  (3)..(3)
<223>  N is a 2',6'-dimethylazobenzene-D-threoninol residue

<220>
<221>  misc_feature
<222>  (7)..(7)
<223>  N is a 2',6'-dimethylazobenzene-D-threoninol residue

<220>
<221>  misc_feature
<222>  (11)..(11)
<223>  N is a 2',6'-dimethylazobenzene-D-threoninol residue

<220>
<221>  misc_feature
<222>  (15)..(15)
<223>  N is a 2',6'-dimethylazobenzene-D-threoninol residue

<400>  2
gcngcgnctc ngcgncgatt attacgcgcg agcgcgc                                 37


<210>  3
<211>  40

<212>  DNA
<213>  artificial

<220>
<223>  aptamer

<400>  3
tggatgatag ctcggtcggg gtggatgggt tggcaagtct                              40


<210>  4
<211>  41
<212>  DNA
<213>  artificial

<220>
<223>  nucleic acid motif



<220>
<221>  misc_feature
<222>  (7)..(7)
<223>  N is a 2',6'-dimethylazobenzene-D-threoninol residue

<220>
<221>  misc_feature
<222>  (11)..(11)
<223>  N is a 2',6'-dimethylazobenzene-D-threoninol residue

<220>
<221>  misc_feature
<222>  (15)..(15)
<223>  N is a 2',6'-dimethylazobenzene-D-threoninol residue

<220>
<221>  misc_feature
<222>  (19)..(19)
<223>  N is a 2',6'-dimethylazobenzene-D-threoninol residue

<400>  4
uuuugcngcg nctcngcgnc gattattacg cgcgagcgcg c                            41